(19) Europäisches Patentamt
European Patent Office
Office européen des brevets

(11) Publication number: **0 413 415 A1**

(12) **EUROPEAN PATENT APPLICATION**

(21) Application number: **90306306.3**

(22) Date of filing: **08.06.90**

(51) Int. Cl.5: **C07C 65/24**, C07D 307/89, C08G 73/10, B01D 71/64

A request for correction of claim 9 has been filed pursuant to Rule 88 EPC. A decision on the request will be taken during the proceedings before the Examining Division (Guidelines for Examination in the EPO, A-V, 2.2).

(30) Priority: **13.06.89 GB 8913589**
**11.10.89 GB 8922882**

(43) Date of publication of application:
**20.02.91 Bulletin 91/08**

(84) Designated Contracting States:
**DE FR GB IT NL**

(71) Applicant: **The British Petroleum Company p.l.c.**
**Britannic House Moor Lane**
**London EC2Y 9BU(GB)**

(72) Inventor: **Eastmond, Geoffrey Charles**
**The University of Liverpool, Dep. of Chemistry**
**PO Box 147, Liverpool L69 3BX(GB)**
Inventor: **Paprotny, Jerzy**
**The University of Liverpool, Dep. of Chemistry**
**PO Box 147, Liverpool L69 3BX(GB)**
Inventor: **Page, Philip Charles Bulman**
**The University of Liverpool, Dep. of Chemistry**
**PO Box 147, Liverpool L69 3BX(GB)**
Inventor: **Richards, Robin Edward**
**1333 Neshaminy Valley Drive**
**Bensalem, Pennsylvania 19020(US)**

(74) Representative: **Dodding, Robert Anthony et al**
**BP INTERNATIONAL LIMITED Patents Division Chertsey Road**
**Sunbury-on-Thames Middlesex, TW16 7LN(GB)**

(54) **Carboxylic acids and derivates thereof.**

(57) Carboxylic acids and derivatives having a general formula X-O-Ar-O-Y in which X and Y are aromatic mono or di-basic carboxylic acid groups of derivatives which may be the same or different. Ar is a divalent aromatic group having at least one tertiary alkyl substituent and not more than one such substituent ortho to each of its bonds to the ether groups. A polyetherimide is prepared by reacting an anhydride derivative of the above formula with a diamine. Gas separation membranes can be fabricated from the resultant polyetherimides.

EP 0 413 415 A1

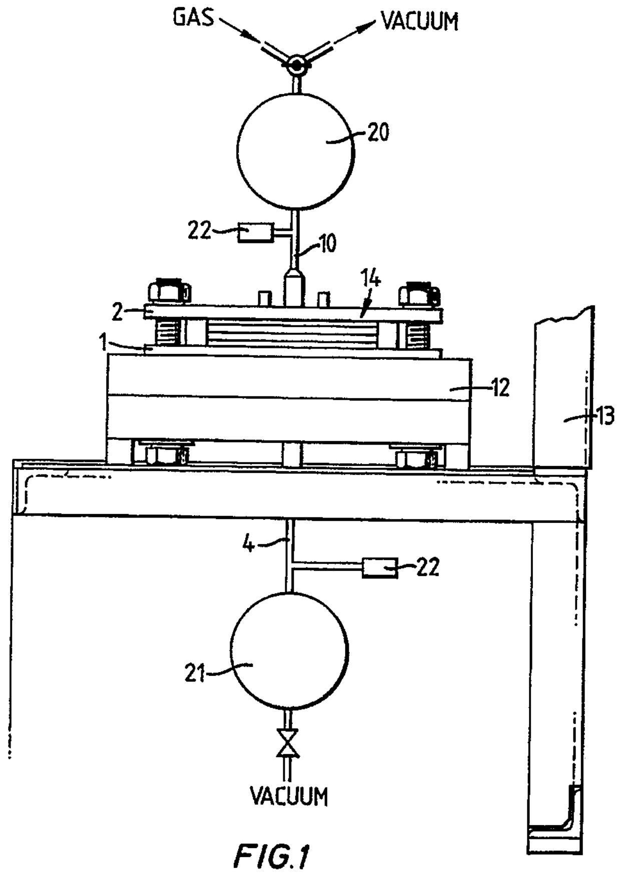


FIG.1

## CARBOXYLIC ACIDS AND DERIVATIVES THEREOF

The present invention relates to a novel class of carboxylic acids and derivatives thereof and in particular to tertiary alkyl substituted bis ether carboxylic acids their esters, acid chlorides and anhydrides and to polymers derived therefrom and to uses for such polymers. The present invention also relates to methods for making these compounds.

Alkyl substituted carboxylic acids are described for example in United States patent number US 3,905,942 which describes the preparation of thermoplastic polyetherimide by reacting an organic diamine with an aromatic bis (ethercarboxylic) acid having the general formula:

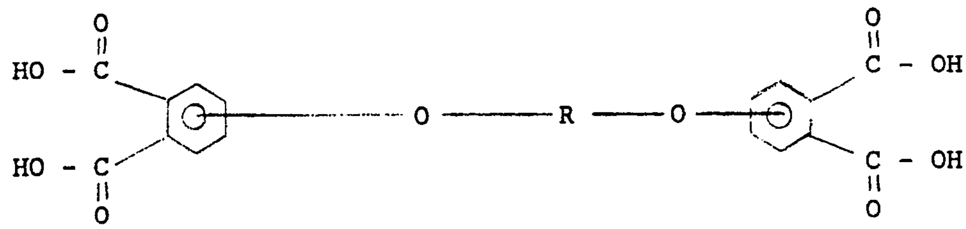

where -R- is selected from the class consisting of a) divalent organic radicals of the formulas,

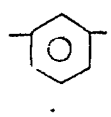 , 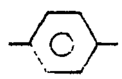 , 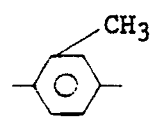 , 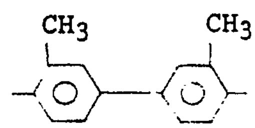

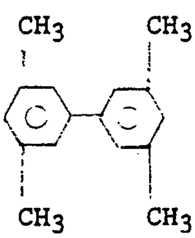 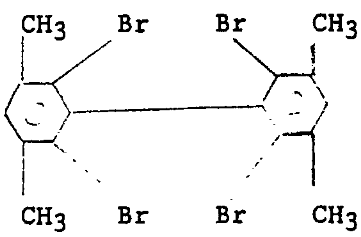 and 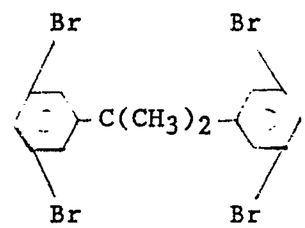

and b) divalent organic radicals included by the formula:

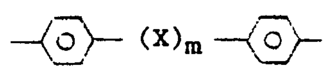

as thereindefined.

United States patent number US 4,078,142 describes an amino-substituted ester of 4-t-butylphthalic anhydride having the general formula:

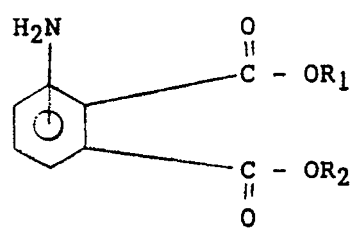

where $R_1$ and $R_2$ are individually selected from the group consisting of alkyl groups and aryl groups.

Stevenson and Mulvaney in Journal of Polymer Science Part A-1 volume 10 pages 2713-2725 (1972) describe preparation of aromatic polyesters of 3,5-di-tert-butyl-4-hydroxybenzoic acid and 3,5-diisopropyl-4-hydroxybenzoic acid.

Polyetherimides derived from alkyl substituted carboxylic acids are known which have etherimide units with the general formula:

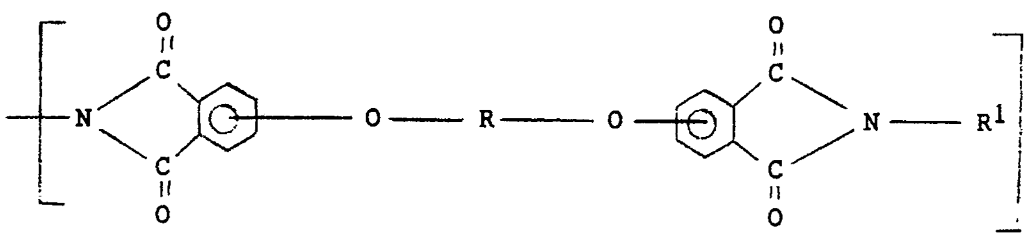

Thus in United States patent numbers 3,847,867, 3,983,093, 3,905,942, 4,017,511 and 4,540,748, -R- may be

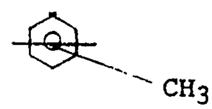

Also in United States patent number 4,717,394, -R- may be

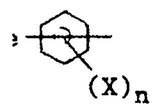

where n is 0 to 4 and -X is an alkyl group having between 1 and 6 carbon atoms, preferably methyl or ethyl or an aromatic group of 6 to 13 carbon atoms. In PCT patent application number WO85/01509, -R- may be a substituted aromatic radical such as

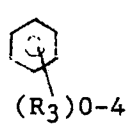

where $-R_3$ is independently $C_1$ to $C_6$ alkyl, aryl or halogen. In United States patent number 4,717,393, -R- may be

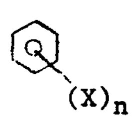

where n = 0 to 4 and -X is independently a primary or secondary alkyl group having 1 to 6 carbon atoms, preferably methyl or ethyl.

There remains a need for tertiary alkyl substituted bis ether carboxylic acids and derivatives thereof.

Thus, according to the present invention there is provided a compound having the general formula:

X — O — Ar — O — Y (I)

in which -X and -Y are aromatic mono- or di-basic carboxylic acid groups or derivatives thereof and may be the same or different, and -Ar- is a divalent aromatic group having at least one tertiary alkyl substituent and not more than one such substituent ortho to each of its bonds to the ether groups of the compound.

-X and -Y may have a general formula selected from the group consisting of:

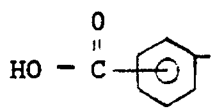 , 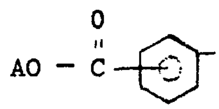 , 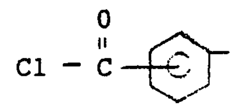

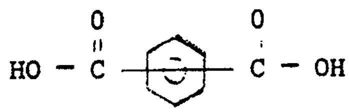 and 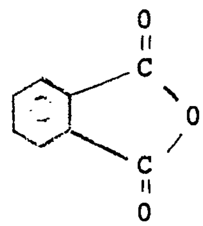

where -A is an alkyl or aryl group having up to 12 carbon atoms.

-Ar- may have a general formula selected from the group consisting of:

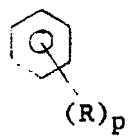 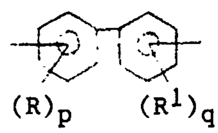 and 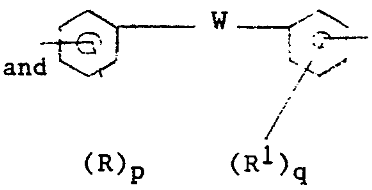

in which -R and -$R^1$ are independently tertiary alkyl groups having up to eight carbon atoms, p and q are independently 0,1,2 or 3, preferably 1 or 2 and p + q is at least one, and -W- is -$CH_2$-, -$C(CH_3)_2$-, -$C(CF_3)_2$- or

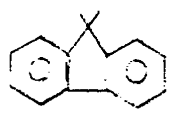

with the proviso that -Ar-does not have more than one tertiary alkyl substituent ortho to each of its bonds to the ether groups of the compound.

-R and -$R^1$ may be tertiary hexyl (-$C(CH_3)_2$-$(CH_2)_2$-$CH_3$) or tertiary pentyl, but are preferably tertiary butyl. Different -R or -$R^1$ groups may be present.

Preferably, -Ar- has the general formula

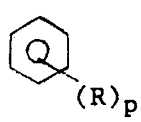

with bonds to the ether groups of the compound which are meta or para to each other.

Also according to the present invention there is provided a polymer comprising etherimide units having the general formula:

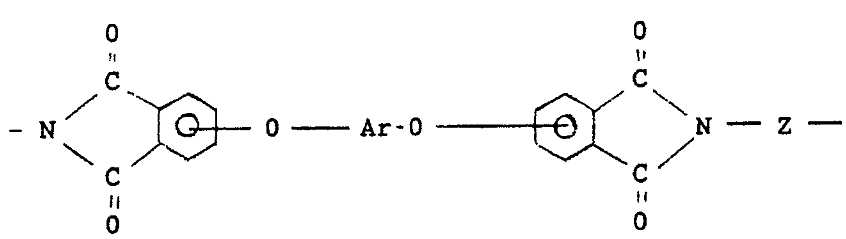 (II)

in which -Ar- is as hereinbeforedefined for general formula (I) and -Z- is an aromatic divalent group.

-Z- may have a general formula selected from the group consisting of:

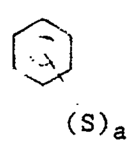

and

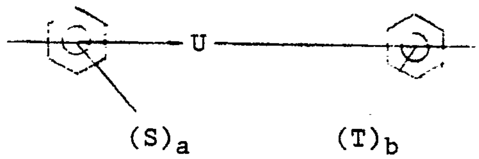

where -S and -T are independently selected from the group consistingof -Cl, -Br, an alkyl group having 1 to 6 carbon atoms and a fluoroalkyl group having 1 to 6 carbon atoms; -S and -T may be the same or different; a and b are independently 0,1,2,3 or 4, for a or b greater than 1 different -S or -T groups may be present in -Z-; and -U- is selected from the group consisting of -O-, -CO-, -CHOH-, -S-, $-SO_2-$. $-CH_2-$, -C-$(CH_3)$-, $-C(CF_3)_2-$ and

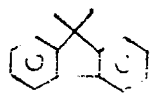

Different etherimide units having the same general formula (I) may be present in the polymer. The polymer may also have etherimide units other than as herein described.

Also according to the present invention there is provided a polymer comprising amide units having general formula:

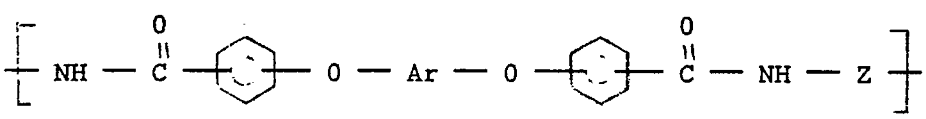

(III)

in which -Ar- is herein defined for general formula (I) and -Z- is as herein defined for general formula (II).

Different amide units having the same general formula (III) may be present in the polymer. The polymer may also have amide units other than as herein described.

Also according to the present invention there is provided a polymer comprising ester units having the general formula:

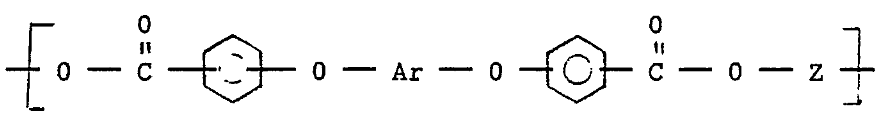

(IV)

in which -Ar- is as herein defined for general formula (I) and -Z-is as herein defined for general formula (II).

Different ester units having the general formula (IV) may be present in the polymer. The polymer may also have ester units other than as herein described.

Also according to the present invention there is provided a method of making a polymer comprising etherimide units having general formula (II) as herein defined, the method comprising reacting a compound having the general formula $H_2N-Z-NH_2$ with a compound having the general formula:

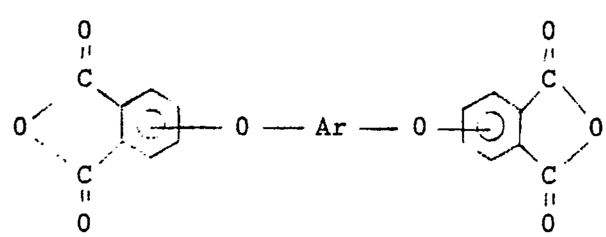

(V)

in which -Z- is as herein defined for general formula (II) and -Ar-is as herein defined for general formula (1).

A mixture of compounds with general formula $H_2N-Z-NH_2$ may be used. The reaction may be performed at an elevated temperature. The reaction may be performed in dimethylacetamide in the

presence of pyridine and acetic anhydride, to convert the intermediate polyamic acid to polyimide. It is desirable to use a solvent in which the polyamic acid is soluble and in which the polyimide remains in solution or at least as a swollen gel after imidisation in the presence of acetic anhydride and pyridine. Also desirably the solvent should not react with the amide or imide linkages and hence be deleterious to polymer formation. Some polar solvents are suitable for the process.

Also according to the present invention there is provided a method of making a polymer comprising amide units having general formula (III) as herein defined, the method comprising reacting a compound having the general formula:

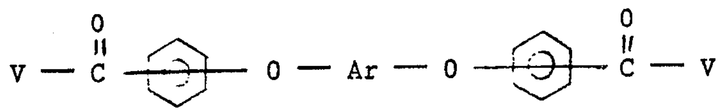

(VI)

in which -Z- is as herein defined for general formula (II) -Ar- is as herein defined for general formula (I) and -V is -OH, -OA, -Cl and -A is as herein defined for general formula (I).

Also according to the present invention there is provided a method of making a polymer comprising ester units having general formula (IV) as herein defined the method comprising reacting together a compound having the general formula HO-Z-OH in which -Z-is as herein defined for general formula (II), with a compound having the general formula (VI) as herein defined.

Also according to the present invention there is provided a method of making a compound having the general formula (V) as herein defined, the method comprising reacting a compound having the general formula:

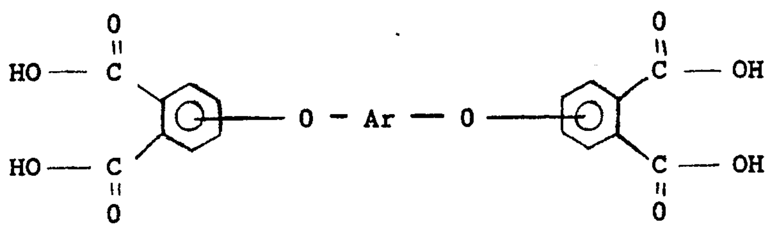

(VII)

in which -Ar- is as herein defined for general formula (V), with acetic anhydride at elevated temperature.

The product of this reaction may be recrystallised with acetic anhydride.

The compound having general formula (VII) may be prepared by hydrolysis of a compound having the general formula:

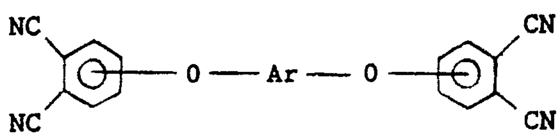

(VIII)

in which -Ar- is as herein defined for general formula (VII). The hydrolysis may be performed by refluxing the compound with a mixture of potassium hydroxide, water and methanol.

The compound having a general formula (VIII) may be prepared by reacting a compound having the general formula:

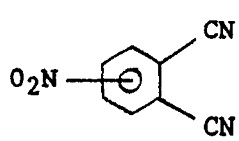

(IX)

with a compound having the general formula HO-Ar-OH in which -Ar- is as herein defined for general formula (VIII).

Preferably the reaction is performed in anhydrous dimethyl sulphoxide in the absence of oxygen and in the presence of potassium carbonate.

The compound having general formula (IX) may be prepared by reacting the corresponding 3- or 4-

nitrophthalic amide with trifluoroacetic anhydride in the presence of 1,4 dioxane and dry pyridine. The 3- or 4- nitrophthalic amide may in turn be prepared by reacting 3- or 4- nitrophthalic acid imide with concentrated (33%w/w) aqueous ammonia solution. 3 and 4-nitrophthalonitrile are also available commerically.

Also according to the present invention there is provided a gas separation membrane comprising a polymer comprising etherimide units as herein described. The membrane may comprise a blend of polymers comprising etherimide units as herein described. The membrane may be a dense or asymmetric film or a hollow fibre membrane.

Also, according to the present invention there is provided a process for separating gases, the process comprisng passing a mixture of two or more gases through a gas separation membrane as herein described, whilst maintaining a differential pressure across the membrane, whereby the gases of the gas mixture are separated by their relative permeation rates through the membrane. By gas it is intended to include vapours. Also according to the present invention there is provided an apparatus for separating gases comprising a gas separation membrane as herein described, means for passing a mixture of two or more gases through said membrane, and means for maintaining a differential pressure across the membrane, whereby gases of the gas mixture are separated by their relative permeation rates through the membrane. The gas separation apparatus and process according to the present invention may be used to separate carbon dioxide/methane mixtures; oxygen/nitrogen mixtures; helium/methane mixtures; carbon monoxide/methane mixtures; hydrogen/methane mixtures and the like. The gas separation apparatus and process of the present invention may also be used for dehydration and for the removal of hydrogen sulphide gas from natural gas and the like.

Polymers comprising amide units according to the present invention may be used for gas separation membranes, structural materials, fibres and the like.

Polymers comprising ester units according to the present invention may be used for gas separation membranes, structural materials and the like.

The invention will now be described by way of example only and with reference to the drawings. Figures 1 and 2 represent in side elevation and cross-section respectively, apparatus used to measure the permeability of membranes according to the present invention. Figure 3 represents in schematic form the apparatus used to measure water vapour permeabilities of polymers prepared according to the present invention.

Example 1 Preparation of 4-nitrophthalic amide

25 g of 4-nitrophthalic acid imide (supplied by Lancaster Synthesis) was suspended in 140 ml of concentrated aqueous ammonia solution (33% $^{w}/_{w}$) and allowed to react at room temperature for six hours before being left in a refrigerator overnight. The cold solid precipitate which was produced was filtered, washed with diethyl ether and dried. A product yield of 25.1 g was obtained. This preparation is described by R.A. McClelland, et al Can. J. Chem, 1985 63 121.

Example 2 Preparation of 3-nitropthalic amide

Example 1 was repeated using 3-nitrophthalic acid imide (supplied by Lancaster Synthesis) in place of the 4-nitrophthalic acid imide.

Example 3 Preparation of 4-nitrophthalonitrile

25g of 4-nitrophthalic amide prepared according to Example 1 was added to 210 ml dry dioxane (supplied by Aldrich) and the mixture was cooled to 0° C. 42 ml of trifluoroacetic anhydride was slowly added to the mixture over a period of three hours. The mixture was then allowed to warm to room temperature whilst being stirred and a clear solution was formed. The mixture was diluted with 700 ml of water and extracted four times with 200 ml aliquots of ethyl acetate. The extracts were combined and washed successively with water, dilute hydrochloric acid (10% $^{v}/_{v}$), water and 5% $^{w}/_{v}$ aqueous sodium chloride solution. The solvent was evaporated on a rotary evaporator and the resultant solid product was recrystallised from 50/70 ($^{v}/_{v}$) acetone/hexane mixture to yield 14 g of 4-nitrophthalonitrile crystals. Further evaporation of the mother liquor and recrystallisation increased the total yield to 79.9%. Theoretical analysis

of $C_8H_3N_3O_2$: C,55.49%, H,1.73%; N,24.27%; found: C,55.32%; H,1.63%; N,24.18%. This preparation method is described by T.W.Hall et al in Nouveau J.de Chimie, 1982 6 653.

Example 4 Preparation of 3-nitrophthalonitrile

Example 3 was repeated using 3-nitrophthalic amide prepared according to Example 2 in place of 4-nitrophthalic amide.

Example 5 Preparation of 1,4-bis-(3,4-dicyanophenoxy)-2,5-di-tert-butyl benzene

17.38 g (0.1 moles) of 4-nitrophthalonitrile, prepared according to Example 3, was dissolved in 150 ml anhydrous dimethyl sulphoxide (DMSO) in a 250 ml three-necked flask fitted with a stirrer, nitrogen-gas inlet and thermometer. 11.1 g (0.05 moles) of 2,5-di-tert-butyl hydroquinone (supplied by Aldrich) was added to the mixture followed by 20 g of anhydrous potassium carbonate. The mixture was stirred at room temperature with a stream of dry, oxygen-free (white spot) nitrogen passing through the flask for 24 hours. The reaction mixture was then poured into 1200 ml of water to produce a solid product which was washed five times with water and three times with methanol. The product (20 g, 85% yield) in the form of a white powder was recrystallised from acetonitrile to yield white crystals of 1,4-bis-(3,4-dicyanophenoxy)-2, 5-di-tert-butylbenzene having the formula:

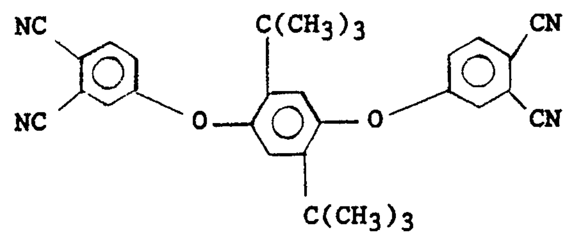

The melting point of this product was above 300°C. Theoretical analysis of $C_{30}H_{26}N_4O_2$: C,75.94%; H,5.48%; N,11.81%; found: C,76.05%; H,5.50%; N,11.89%.

Example 6 Preparation of 1,4-bis-(2,3-dicyanophenoxy)-2,5-di-tert-butyl benzene

The same apparatus as was used in Example 5 was used in this example. 8.8 g (0.051 moles) of 3-nitrophthalonitrile, prepared according to Example 4, was dissolved in 75 ml of anhydrous dimethyl sulphoxide in the three-necked flask. 5.55 g (0.025 moles) of 2,5-di-tert-butyl hydroquinone and 10 g of potassium carbonate were added to the reaction mixture. The mixture was stirred at room temperature with a stream of dry, oxygen-free nitrogen passing through the flask for 26 hours. The product was isolated by pouring the reaction mixture into 500 ml of water. The resultant solid precipitate was filtered and washed until the effluent was neutral. The product was further washed with boiling methanol until it became a greyish-white powder 10.6 g (90.2% yield). After recrystallisation from boiling acetonitrile white crystals of 1,4-bis-(2,3-dicyanophenoxy)-2,5-di-tert-butyl benzene were obtained having the formula:

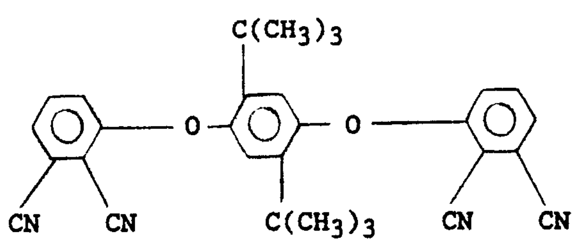

The melting point of this product was above 300°C. Theoretical analysis of $C_{30}H_{26}N_4O_2$ : C,75.94%; H,5.48%; N,11.81%; found: C,75.61%; H,5.48%; N,11.98%.

Example 7 Preparation of 1,4-bis-(3,4-dicarboxyphenoxy)-2,5-di-tert-butyl benzene

25 g of potassium hydroxide was dissolved in 40 g of water in a 250 ml round-bottomed flask fitted with a reflux condenser. 19 g of 1,4-bis-(3,4-dicyanophenoxy)-2,5-di- tert-butyl benzene, prepared according to Example 5, was added to the heated solution in the flask, followed by 100 ml of methanol. The mixture was refluxed for 40 hours at the end of which time evolution of ammonia had ceased. The mixture was then poured into a beaker and diluted with water to a total volume of 300 ml. The acidity was adjusted, by addition of concentrated hydrochloric acid solution, to a pH of between 1.5 and 2. The resultant solid precipitate was filtered, washed three times with water and dried to yield 21.6 g (98% yield) of 1,4-bis-(3,4-dicarboxyphenoxy)-2,5-di- tert-butyl benzene having the formula:

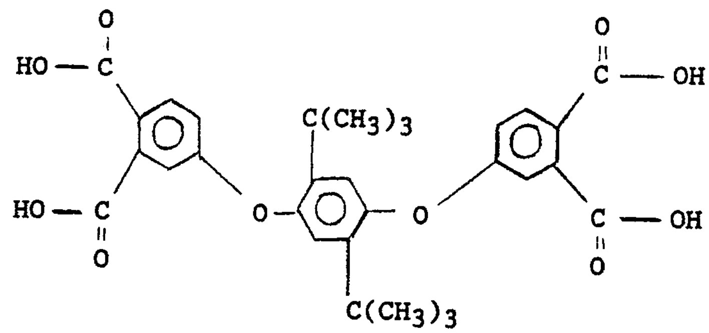

This had a melting point in the range 210 to 220° C. The product was not purified further but was used in Example 8 below.

Example 8 Preparation of 1,4-bis-(3,4-dicarboxyphenoxy)-2,5-di-tert-butyl benzene dianhydride

20 g of the 1,4-bis-(3,4-dicarboxyphenoxy)-2,5-di-tert-butyl benzene prepared according to Example 7 was dissolved in 200 ml of glacial acetic acid and 150 ml of acetic anhydride in a 500 ml round-bottomed flask. The mixture was refluxed for 2 hours. The resultant solid product which crystallised on cooling was filtered and recrystallised from 200 ml of acetic anhydride to give a 90% yield of 1,4-bis-(3,4-dicarboxyphenoxy)-2,5-di-tert-butyl benzene dianhydride having the formula:

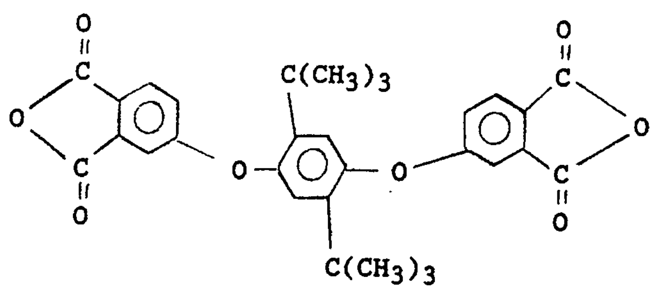

This had a melting point of 249 to 250° C. Theoretical analysis of $C_{30}H_{26}O_8$: C,70.04%; H,5.06%; N,0%; found: C,69.85%, H,5.08%, N,1.2 x $10^{-4}$%.

Example 9 Preparation of 1,4-bis-(2,3-dicarboxyphenoxy)-2,5-di-tert- butyl benzene dianhydride

The tetranitrile prepared according to Example 6 was converted to the corresponding tetraacid using an equivalent preparation to that of Example 7. The tetraacid was then converted to the corresponding bisetheranhydride, 1,4-bis-(2,3-dicarboxyphenoxy)2,5-di-tert butyl benzene dianhydride using an equivalent preparation to that in Example 8. This bisetheranhydride has the formula:

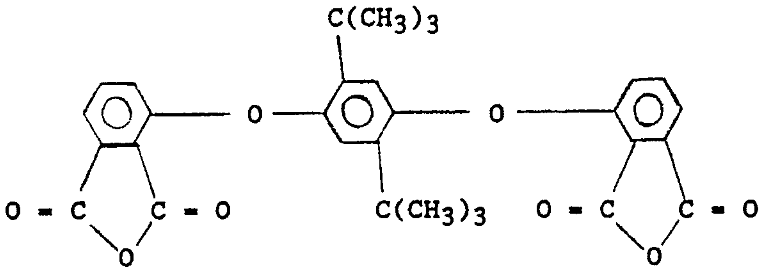

This bisetheranhydride was in the form of pale yellow crystals with a melting point above 300 °C. Theoretical analysis of $C_{30}H_{26}O_8$ : C, 70.03%, H, 5.05%; found : C, 69.85%, H, 5.08%.

Example 10 Preparation of polymer from 4,4'-diaminodiphenylether and 1,4-bis-(3,4-dicarboxyphenoxy)-2,5-di-tert-butyl benzene dianhydride

2.00 g (0.01 mole) of 4,4'-diaminodiphenylether (supplied by Aldrich) was dissolved in 50 ml of anhydrous dimethylacetamide (DMAC) (supplied by Aldrich) in a flask fitted with a magnetic stirrer. 5.14 g (0.01 mole) of 1,4-bis-(3,4-dicarboxyphenoxy)-2,5-di-tert-butyl benzene dianhydride was added in one portion at room temperature. Within four hours the mixture became very viscous and impossible to stir with the laboratory magnetic stirrer used. To this mixture was added 5 g of anhydrous pyridine and 5 g of acetic anhydride. The mixture became opaque after two hours; after standing for 12 hours it had formed a gel which was washed three times with methanol, dried, and dissolved in dichloromethane. After precipitating into methanol and drying the yield of polymer was 6.7 g.

Example 11 Preparation of polymer from 4,4'- diaminodiphenylether and 1,4-bis-(2,3-dicarboxyphenoxy)-2,5-di- tert-butyl benzene dianhydride

1.600 g (0.008 mole) of 4,4'-diaminodiphenylether (supplied by Adrich) was dissolved in 50 ml of anhydrous dimethylacetamide (DMAC) (supplied by Aldrich) in a flask fitted with a magnetic stirrer. 4.112 g of 1,4-bis-(2,3-dicarboxyphenoxy)-2,5-di-tert-butyl benzene dianhydride was added with stirring at room temperature. After 20 hours the mixture had become very viscous. To this mixture was added 5 g of anhydrous pyridine and 5 g of acetic anhydride. After standing for six hours the polymer was precipitated into methanol and dried. The yield of polymer was 5.4 g.

Example 12 Preparation of polymer from 1,4-bis-(3,4-dicarboxyphenoxy)- 2,5-di-tert butyl benzene dianhydride and a mixture of 2,2'-bis(4-aminophenyl)propane, and 4,4'-diaminodiphenylether

1 g (0.005 mole) of 4,4' diaminodiphenylether (supplied by Aldrich) and 1.13 g (0.005 mole) of 2,2-bis-(4-aminophenyl)propane was dissolved in 50 g of dry dimethylacetamide in a flask fitted with a magnetic stirrer. The 2,2-bis-(4-aminophenyl) propane was prepared by the method described by H.Krimm et al in German patent number DE 1220863 by reacting aniline hydrochloride with acetone in appropriate stoichiometric amounts in a sealed glass vessel at 145 °C for seven days (yield 55%). 5.14 g (0.01 mole) of 1,4-bis-(3,4-dicarboxyphenoxy)-2,5-di-tert-butyl benzene dianhydride was added to the flask containing the two diamines. The solution became extremely viscous within two hours. A mixture of 5 g pyridine and 5g acetic anhydride was added to the flask and the mixture was left overnight to form an opaque solid gel. The gel was washed with methanol and the product dried. The dry polymer was redissolved in 70 ml of dichloromethane and reprecipitated into methanol, filtered and dried. Yield of polymer was over 6.5g.

Example 13 Preparation of polymer from 1,4-bis-(3,4-dicarboxyphenoxy)-2,5-di-tert butyl benzene dianhydride and 3,3',5,5'-tetramethylbenzidene

3,3',5,5'- tetramethylbenzidene (0.240g, 1mmol)(supplied by Aldrich) having the formula

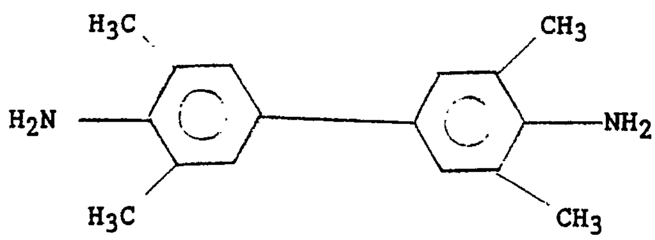

was dissolved in dry dimethylacetamide (DMAC) (5ml) in a flask fitted with a magnetic stirrer, and 1,4-bis-(3,4-dicarboxyphenoxy)-2,5-di-tert-butyl benzene dianhydride (BHDA) (0.514g, 1mmol) prepared according to Example 8 added. The reaction mixture was stirred at 50 °C for 60 minutes and then kept at room temperature for a further 60 hours. Imidisation was carried out by addition of pyridine (0.7g) and acetic anhydride (0.7g) followed by standing for 3 hours at room temperature; after 15 minutes the mixture had become a solid gel. The mixture was precipitated into methanol, washed with methanol, dried, and redissolved in chloroform. Reprecipitation into methanol gave, after washing and drying, 0.70g of lemon-coloured polymer.

Example 14 Preparation of polymer from 1,4-bis-(3,4-dicarboxyphenoxy)-2,5-di-tert butyl benzene dianhydride and 3,3′,5,5′-tetramethyl-4,4′-diaminodiphenylmethane

3,3′,5,5′-Tetramethyl-4,4′-diaminodiphenylmethane (0.254g, Immol) (development product supplied by Seika and Co) having the formula

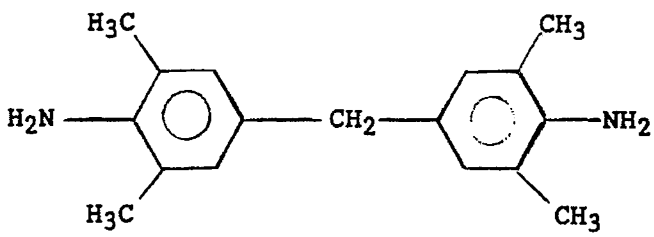

was dissolved in DMAC (5ml) in a flask fitted with a magnetic stirrer, and 1,4-bis-(3,4-dicarboxyphenoxy)-2,5-di-tert butyl benzene dianhydride (BHDA) (0.514g, Immol) prepared according to Example was 8 added. The reaction mixture was stirred at 70 °C for 75 minutes; the mixture becoming clear after 60 minutes, and then was kept at room temperature for a further 48 hours. Imidisation was carried out by addition of pyridine (0.7g) and acetic anhydride (0.7g) followed by standing for 3 hours at room temperature. The lemon coloured solution was precipitated into methanol, and the crude product washed, dried, and redissolved in chloroform. Reprecipitation into methanol gave, after washing and drying, 0.73g of polymer.

Example 15 Preparation of polymer from 1,4-bis-(3,4-dicarboxyphenoxy)-2,5-di-tert butyl benzene dianhydride and 3,3′,5,5′-tetraethyl-4,4′-diaminodiphenylmethane

3,3′,5,5′-Tetraethyl-4,4′-diaminodiphenylmethane (development product supplied by Lonza) having the formula

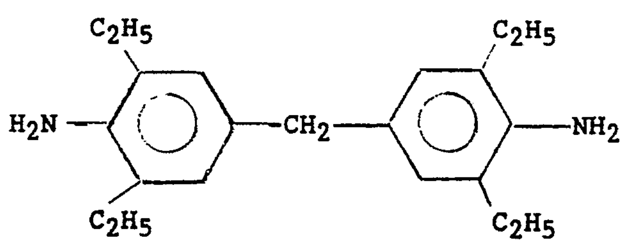

(2.48g, 8mmol) was dissolved in DMAC (40 ml in a flask fitted with a magnetic stirrer, and 1,4-bis-(3,4-dicarboxyphenoxy)-2,5-di-tert butyl benzene dianhydride (BHDA) (4.112g,8mmol) prepared according to Example 8 was added. The reaction mixture was stirred at 60 °C for 10 hours. Imidisation was carried out by addition of pyridine (5g) and acetic anhydride (5g) followed by standing for 2 hours at 60 °C. The crude product was precipitated into methanol, dried, and redissolved in chloroform. Reprecipitation into methanol gave, after washing and drying, 6.4g of pale yellow polymer.

Example 16 Preparation of 1,4-bis-3,4-dicyanophenoxy)-2,5-di-tert-amyl benzene

4-Nitrophthalo-nitrile (8.7g, 50mmol) was dissolved in anhydrous DMSO (50ml) under a nitrogen atmosphere and 2,5-di-tert-amyl-hydroquinone having the formula:

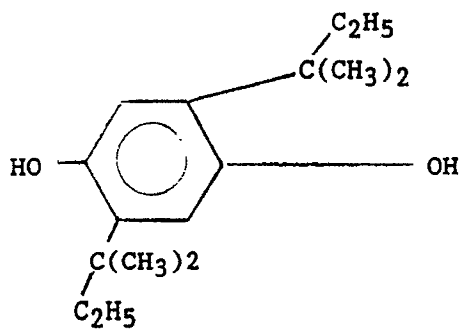

(6.25g, 25mmol) was added. After the material had dissolved, anhydrous potassium carbonate (10g) was added, and the reaction mixture stirred at 60-70 °C for two hours and then at room temperature for 20 hours. A nitrogen atmosphere was maintained throughout. The reaction mixture was poured into water (600ml) and the precipitate collected and washed with water until the washings reached neutral pH. The precipitate was washed with methanol at room temperature and then treated with boiling methanol, filtered while hot, and dried to give 1,4-bis-(3,4-dicyanophenoxy)-2,5-di-tert-amyl benzene having the formula:

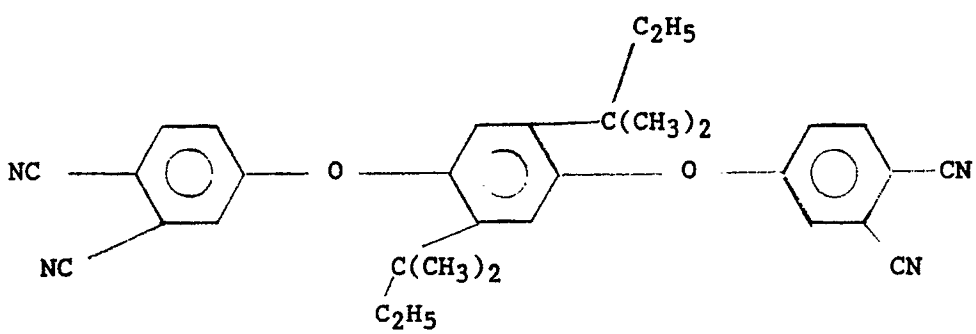

(9.0g 75%) (found: C, 75.80; H, 5.84; N. 11.34. Calc for $C_{32}H_{30}N_4O_2$:C 76.50:H 6.00; N,11.10%)

Example 17 Preparation of 1,4-bis-(3,4-dicarboxyphenoxy)-2,5-di-tert-amyl benzene

Potassium hydroxide (10g, 0.178 mmol) was added to water (10ml) followed by 1,4-bis-3,4-dicyanophenoxy)-2,5-di-tert-amyl benzene (9g, 17.3 mmol) and methanol (15ml). The mixture was boiled at reflux for 42 hours, diluted with water (150ml), and acidified to pH1 using concentrated hydrochloric acid. The oily solid precipitate was digested using ether (3x100ml) and the combined organic solutions dried over magnesium sulphate and evaporated to give 1,4-bis-(3,4-dicarboxyphenoxy)-2,5-di-tert-amyl benzene having the formula:

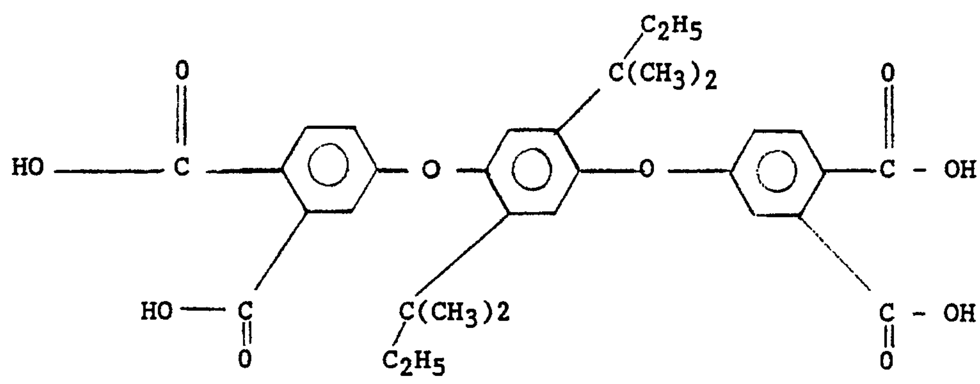

(9.9g, 98%)

Example 18 Preparation of 1,4-bis-(3,4-dicarboxyphenoxy)-2,5-di-tert-amyl benzene dianhydride

1,4-bis-(3,4-dicarboxyphenoxy)-2,5-di-tert-amyl benzene (9.9g, 17.0 mmol) was added to acetic acid (10ml) and acetic anhydride (15ml) and the mixture boiled at reflux for three hours. The mixture was allowed to cool to room temperature causing crystallisation. The supernatant liquid was decanted and the crystalline material dissolved in boiling acetic anydride (15ml). Upon cooling 1,4-bis-(3,4-dicarboxyphenoxy)-2,5-di- tert-amyl benzene dianhydride having the formula:

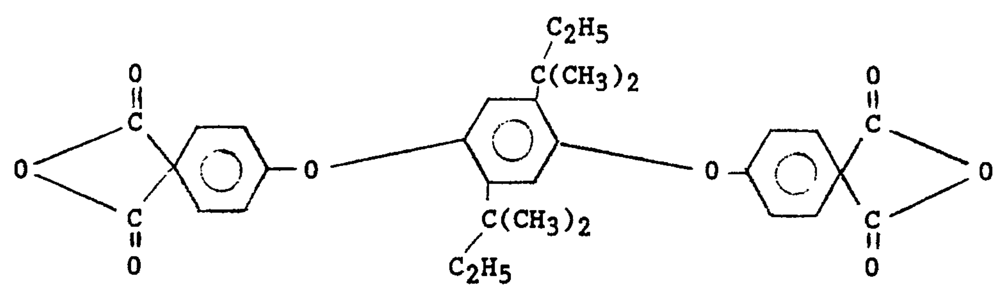

(6.0g 65%) crystallised from the solution. Further product (1.7g, 18%) was recovered from the mother liquors. (found: C, 70.41; H, 5.53 Calc for $C_{32}H_{34}O_8$: C, 70.32: H, 6.21%).

Example 19 Preparation of polymer from 1,4-bis-(3,4-dicarboxyphenoxy)-2,5-di-tert-amyl benzene dianhydride and 3,3′,5,5′-tetramethyl-4,4′-diaminodiphenylmethane

3,3′,5,5′-tetramethyl-4,4′-diaminodiphenylmethane as used in Example 14 (2.54g, 0.01 mol) was dissolved in anhydrous dimethyl acetamide (40ml) and 1,4-bis-(3,4-dicarboxyphenoxy)-2,5-di-tert-amyl benzene dianhydride (5.46g 0.01 mol) was added in one portion. After standing at room temperature for 48 hours the mixture because extremely viscous and was diluted with further dimethyl acetamide (15ml). Pyridine (10ml) and acetic anhydride (10ml) were added and the mixture allowed to stand for 24 hours and then poured into methanol (500ml). The precipitate was collected and dried then dissolved in chloroform and reprecipitated to give polymer product (6.5g).

Example 20 Repeat preparation of polymer from 1,4-bis(3,4-dicarboxyphenoxy)-2,5-di-tert-butyl benzene dianhydride and 3,3′,5,5′-tetramethylbenzidene

3,3′,5,5′-tetramethylbenzidene as used in Example 13 (2.4g, 1 mmol) was dissolved in anhydrous dimethyl acetamide (40ml) and 1,4-bis(3,4- dicarboxyphenoxy)-2, 5-di-tert-butyl benzene dianhydride as prepared in Example 8 was added with stirring; after stirring for one hour at room temperature the mixture was heated to 70° C for 15 minutes, then stirred at room temperature for 72 hours. Acetic anhydride (7 ml)

and pyridine (7 ml) were added and the mixture stirred at room temperature for 12 hours. The resulting yellow gel was washed with methanol, dried redissolved in chloroform, and precipitatated from methanol to give polymer (6.7g).

Example 21 Preparation of a polymer from 3,3′,5,5′-tetramethyl-4,4′-diaminodiphenylmethane and a mixtue of 1,4-bis [3,4-dicarboxyphenoxy]-2,5-di-tert-butyl benzene dianhydride and 3,3′,4,4′-benzophenone tetracarboxylic acid dianhydride.

2.54 g (0.01 mol) of 3,3′,5,5′-tetramethyl-4,4′-diaminodiphenylmethane (development product from Seika and Co, Japan) was dissolved in 40 ml N,N-dimethylacetamide (DMAC) and then 4.112 g (0.008 mol) of 1,4-bis-(3,4-dicarboxyphenoxy)-2,5-di-tertiary-butyl benzene dianhydride was added and the mixture was heated to 50-60°C for 10 min to dissolve the anhydride. Then 0.644 g (0.002 mol) of 3,3′,4,4′-benzophenone tetracarboxylic acid dianhydride was added. The mixture was allowed to react for 24 hours and the resulting polyamic acid was imidized with a mixture of acetic anhydride (5 ml) and pyridine (5 ml). After six hours reaction the product was precipitated into methanol. After drying, the polymer was redissolved in chloroform (70 ml) and reprecipitated into methanol, filtered and dried. The polymer yield was 7 g (almost theoretical). The whole of the synthesis and work-up were carried out in a laboratory illuminated with inactive sodium light.

Example 22 Preparation of 4,4′-bis[3,4-dicyanophenoxy]-3,3′-di-tert-butyl-1,1′-biphenyl.

4-nitrophalonitrile (19.03 g, 0.1 mol plus 10% excess) was dissolved in anhydrous DMSO (150 ml) under a nitrogen atmosphere and 3,3′-di-tert-butyl-4,4′-biphenol (14.9 g, 0.05 mol) having the formula:

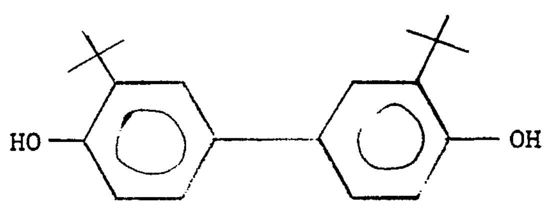

was added. After the materials had dissolved, anhydrous potassium carbonate (22.5 g) was added and the reaction mixture was stirred for 24 hours at room temperature. The reaction mixture was poured into water (1:1) and the precipitate was collected and washed with water until the washings were neutral. The precipitate was then heated to boiling in methanol (200 ml) and filtered off; this procedure was repeated twice. The precipitate was then dried to yield 28.46 g of off-white powder. The crude material was then recrystallized from 600 ml of acetonitrile to yield 24.5 g (89% yeild) of pure tetranitrile having the formula:

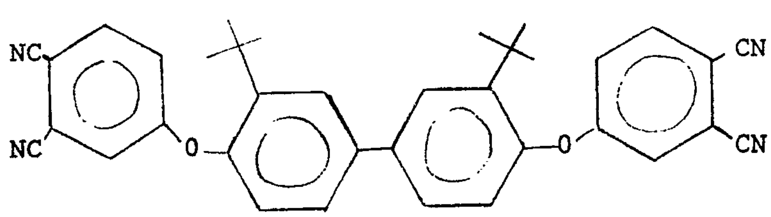

Theoretical analysis of $C_{39}H_{36}N_4O_6$: C,78.54%; H,5.45%; N,10.18%; found: C,78.31%; H,5.63%; N,11.5%.

Example 23 Preparation of 4,4′-bis[3,4-dicarboxyphenoxy]-3,3′-di-tert-butyl-1,1′-biphenyl.

Potassium hydroxide (35 g) was dissolved in water (50 ml) and then 24 g (43.6 mmol) of 4,4′-bis-[3,4-dicyanophenoxy]-3,3′-di-tert-butyl-1,1′-biphenyl, prepared according to Example 22, and 120 ml of methanol were added. The mixture was boiled under reflux for 75 hours and then diluted with water (300 ml) and acidified to pH 0.5 with concentrated hydrochloric acid was filtered off and washed with water until the

washings were neutral. The solid was then dried to yield 24.7 g of 4,4′-bis-[3,4-dicarboxyphenoxy]-3,3′-di-tert-butyl-1,1′-biphenyl having the formula:

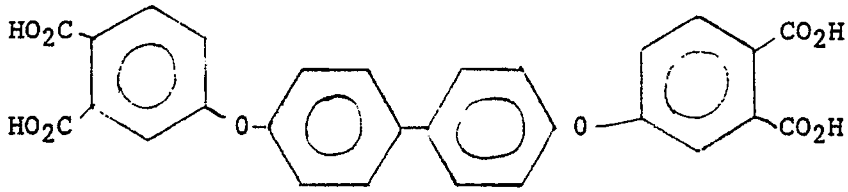

Example 24 Preparation of 4,4′-bis[3,4-dicarboxyphenoxy]-3,3′-di-tert-butyl-1,1′-biphenyl dianhydride.

4,4′-bis-[3,4-carboxyphenoxy]-3,3′-di-tert-butyl-1,1′-biphenyl (24.7 g, 39 mmol), prepared according to example 23, was added to 50 ml of acetic anhydride and 20 ml acetic acid and the mixture was boiled for 3 hours. The mixture was then cooled to room temperature to cause crystallization. The product was filtered off and dried to yield 21.8 g (89.37% yield) of crude bisanhydride which was then recrystallized from 60 ml of boiling acetic anhydride to yield 12.86 g of bisanhydride and an additional 5.3 g of bisanhydride as a second crop, to give a total yield of 18.16 g (78.92%) of bisanhydride having the formula:

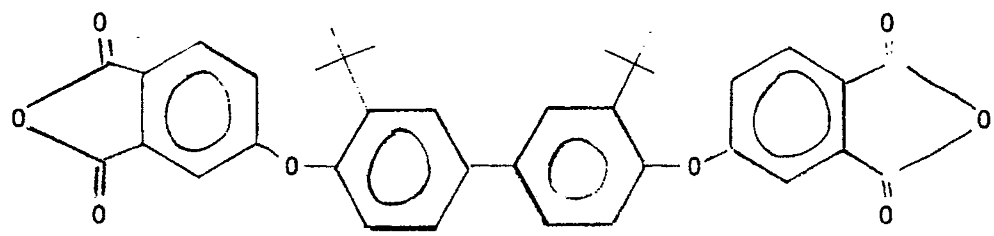

Theoretical analysis of $C_{36}H_{30}O_8$: C,73.22%; H,5.08%: found C,73.6%; H,5.17%.

Although the above procedure generally gave bisanhydrides of sufficient purity to provide high molecular weight polymers, in the case of the above anhydride, even after a further three recrystallizations, the polymers subsequently prepared had peak molecular weights of only 18-22 kg $mol^{-1}$. To obtain polymers with very high molecular weight additional recrystallization from acetonitrile (80 ml) and acetic anhydride (10 ml) was necessary.

Example 25 Preparation of a polymer from 3,3′,5,5′-tetramethyl-4,4′-diaminodiphenylmethane and 4,4′-bis-[3,4-dicarboxyphenoxy[-3,3′-di-tert-butyl-1,1′-biphenyl dianhydride.

3,3′,5,5′-tetramethyl-4,4′-diaminodiphenylmethane (0.2540 g, 1 mmol) was dissolved in 4 ml DMAC in a flask fitted with a magnetic stirrer. Then 0.5900 g, 1 mmol) of 4,4′-bis-[3,4-dicaroxyphenoxy]-3,3′-di-tert-butyl-1,1′- biphenyl dianhydride, prepared according to Example 24 but subsequently recrystallized from a mixture of acetonitrile (80 ml) and acetic anhydride (10 ml) (see example 24), was added. The solution was allowed to react overnight during which time it became very viscous. The reaction mixture was imidized with 2 ml of 50:50 acetic anhydride/pyridine mixture and kept for 4 hours after which it was precipitated into methanol, washed and dried to yield 0.75g of dry yellow polymer.

Example 26 Preparation of 1,1-bis[4-(3,4-dicyanophenoxy)-2-methyl-5-tert-butyl-phenyl]butane.

4-nitrophalonitrile (8.7 g, 50 mmol) was dissolved in anhydrous DMSO (150 ml) under a nitrogen atmosphere and 4,4′-butylidene-bis-(6-tert-butyl-metacresol) (9.56g, 25 mmol) (supplied by TCI, Tokyo) having the formula:

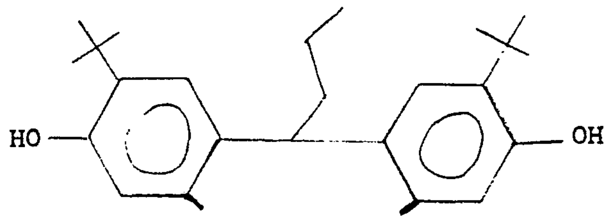

was added. After the materials had dissolved, anhydrous potassium carbonate (10 g) was added and the reaction mixture was stirred for 10 hours at 60 °C and additionally for 14 hours at room temperature. A nitrogen atmosphere was maintained throughout and the flask was fitted with a calcium chloride guard tube. The reaction mixture was poured into water (600 ml) and the precipitate was collected and washed with water until the washings were neutral. The precipiate was then washed with methanol to remove coloured impurities and dried to yield 13.0g (82.27%) of 1,1-bis-[4-(3,4-dicyanophenoxy)-2-methyl-5-tert-butylphenyl]-butane having the formula:

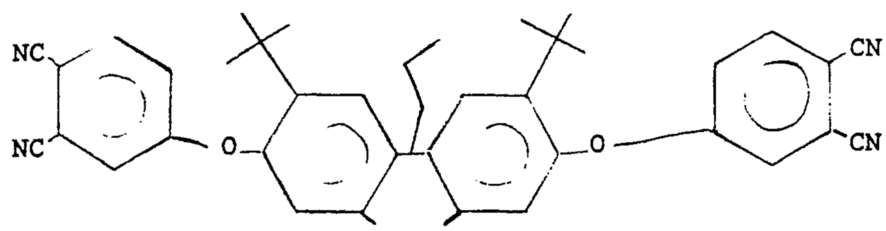

Theoretical analysis of $C_{42}H_{42}N_4O_2$: C,79.5%; H,6.6%; N,8.8%; found: C,79.27%; H,6.76%; N,8.89%.

Example 27 Preparation of 1,1-bis[4-(3,4-dicarboxyphenoxy)-2-methyl-5-tert-butyl-phenyl]butane

Potassium hydroxide (12 g) was dissolved in water (15 ml) and then 12.8 g (20 mmol) of 1,1-bis-[4-(3,4-dicyanophenoxy)-2-methyl-5-tert-butyl-phenyl]butane, prepared according to Example 26, and methanol (30 ml) were added. The mixture was boiled under reflux for 56 hours and then diluted with water and acidified to pH 1 with concentrated hydrochloric acid. The precipitate was filtered off and washed with distilled water until the washings were neutral to give 1,1,-bis=[4-(3,4-dicarboxyphenoxy)-2-methyl-5-tert-butyl-phenyl]-butane, having the formula:

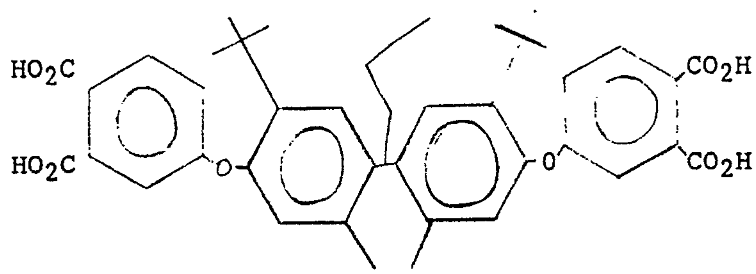

The yield was 13.8g (98%).

Example 28 Preparation of 1,1-bis[4-(3,4-dicarboxyphenoxy)-2-methyl-5-tert-butyl-phenyl]butane dianhydride.

1,1-bis-[4-(3,4-dicarboxyphenoxy)-2-methyl-5-tert-butyl-phenyl] butane (13.8 g, 19 mmol), prepared according to example 27, was added to acetic anhydride (35 ml) and boiled for 1 hour when 15 ml of acetic acid was added and boiling was continued for a further 1.5 hours after which the mixture was allowed to cool. Because no product crystallised out at this stage, half the volume of liquid was distilled off. The

crystals which then formed were filtered off and recrystallized from 20 ml of acetic anhydride, resulting in crystals of 1,1-bis-[4-(3,4-dicarboxyphenoxy)-2-methyl-5-tert-butyl-phenyl]butane dianhydride having the formula:

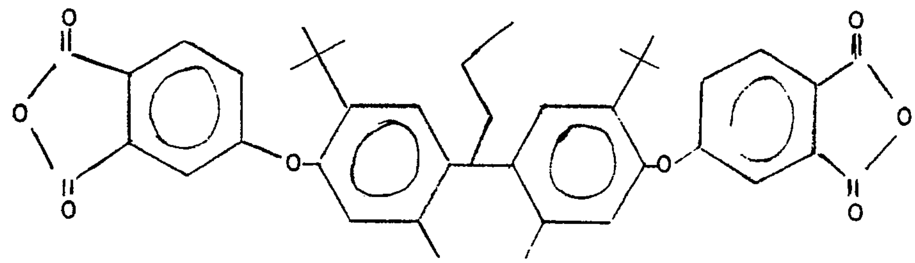

The yield was 9.26 g (72.34%). Theoretical analysis of $C_{42}H_{42}O_8$: C,74.15%; H,6.23%; found: C,74.15%; H,6.23%.

Example 29 Preparation of a polymer from 3,3′,5,5′-tetramethyl-4,4′-diaminodiphenylmethane and 1,1-bis[4-(3,4-dicarboxyphenoxy)-2-methyl-5-tert-butyl-phenyl]butane dianhydride.

3,3′,5,5′-tetramethyl-4,4′-diaminodiphenylmethane (2.032 g, 8 mmol) was dissolved in 50 ml dry DMAC after which 5.392 g (8 mmol) of 1,1-bis-[4-(3,4-dicarboxyphenoxy)-2-methyl-5-tert-butyl-phenyl] butane dianhydride was added. The solution was kept at room temperature for 48 hours, during which period the viscosity increased considerably, after which it was additionally stored at 30°C for 6 hours. The mixture was then imidized with 5 ml of acetic anhydride and 5 ml of pyridine for a period of 5 hours after which the mixture was precipitated into methanol. The yield of polymer, after drying, was 7.1 g.

Example 30 Preparation of 1,4-bis[4-cyanophenoxy]-2,5-di-tert-butyl benzene.

To a three-necked round-bottomed flask equipped with a nitrogen inlet, thermometer, Dean-Stark trap and magnetic bar there was placed 150 ml of anhydrous NMP, 11.2g (0.05 mol) of di-tertiary butylhydroquinone (Aldrich), 13.3g (0.10 mol + 10% excess) of p-fluoro benzonitrile and 14g of anhydrous potassium carbonate. 50 ml of toluene was added through the Dean-Stark trap. The reaction mixture was heated under nitrogen for 10 hours at 130-140°C and then the temperature was raised, by distilling off the toluene, to 170-180°C for an additional 4 hours. The remaining toluene was then distilled off and the temperature rose to 200°C. The reaction mixture was poured into 1.5 l of water. The solid was filtered off and washed with water and three times with methanol to yield a pale grey powder which was recrystallized from 1.3 l of acetone to yield 19g (89.6% yield) of white pearl-like plates.

Elemental analysis for $C_{28}H_{28}N_2O_2$: $C_{calc}$ = 79.24%; $H_{calc}$ = 6.60%; $N_{calc}$ = 6.6%; $C_{found}$ = 79.19%; $H_{found}$ = 6.60%; $N_{found}$ = 6.43%

Example 31 Preparation of 1,4-bis[carboxyphenoxy]-2,5-di-tert-butyl benzene.

The 1,4-bis(4-cyanophenoxy)-2,5-di-tert-butyl benzene prepared according to Example 30 and having the formula:

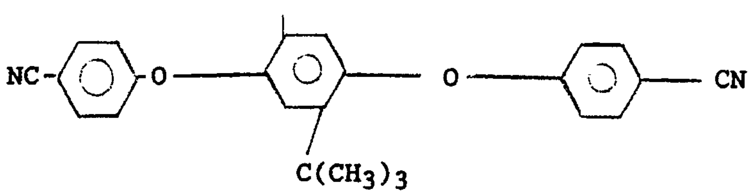

was hydrolysed as follows. 12.0g (0.0235 mol) of the compound was placed in a round-bottomed flask equipped with a magnetic-stirrer bar. A solution of 26g of potassium hydroxide in 40 ml of water was added

together with 100 ml of methanol. The mixture was refluxed for 47 hours and when the evolution of ammonia had ceased. The reaction mixture was then diluted to 1:1 and acidified to pH 0.5, boiled and filtered while hot, washed with distilled water to yield 12.8 g of a snow-white powder. The crude acid was recrystallized from 180ml tetrahydrofuran and 90 ml of acetic acid, filtered and dried to yield 11.0 g of white product (yield was 84.6% of the theoretical) having the formula:

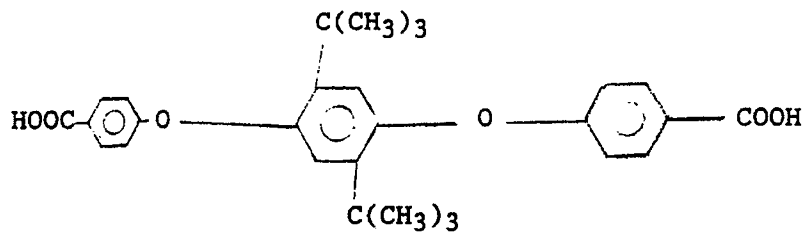

Elemental analysis for $C_{28}H_{30}O_6$ : $C_{calc}$ = 72.7%; $H_{calc}$ = 6.49%; $C_{found}$ = 71.7%; $H_{found}$ = 6.47%.

Example 32 Preparation of a polymer from 3,3′,5,5′-tetramethylbenzidene and 1,4-bis[4-carboxyphenoxy]-2,5-di-tert-butyl benzene.

In 13.7g of n-methyl pyrrolidone (NMP) there was dissolved 0.6 g of anhydrous calcium chloride followed by 0.2 g of anhydrous lithium chloride and 2 ml of anhydrous pyridine. This solution was heated to 95°C when 3,3′,5,5′-tetramethylbenzidene (0.240 g, I mmol) was added followed by 1,4-bis-[4-carboxyphenoxy]-2,5-di-tert-butyl benzene (0.462 g, 1 mmol). Then 2.2 g (7 mmol) of triphenylphosphite was added in two portions. Then, additionally, 1 ml of pyridine and 1.5 ml of triphenyl phosphite were added and the reaction mixture was heated to 150°C for 7 hours. The reaction mixture was then precipitated into an equivolume mixture of methanol and water to yield 0.68 g of a polymer soluble in NMP.

Comparative Experiment A: Preparation of 1,4-bis- (3,4-dicyanophenoxy)-2,3,5-trimethyl benzene

14.0g (0.0809 moles) of 4-nitrophthalonitrile prepared according to Example 3 was dissolved in 120 ml anhydrous dimethyl sulphoxide. 6.08g (0.04 moles) of trimethylhydroquinone (supplied by Aldrich) followed by 15g of anhydrous potassium carbonate was added to the mixture. The mixture was allowed to react with stirring at room temperature under a stream of dry, oxygen-free nitrogen for 24 hours. The reaction mixture was then poured into 500 ml of water. The solid which precipitated was filtered off and washed with water until the effluent was neutral. The product was further washed with boiling methanol until it became white. It was then recrystallised from boiling acetonitrile to give slightly yellow crystals of 1,4-bis-(3,4-dicyanophenoxy)-2,3,5-trimethyl benzene having the formula:

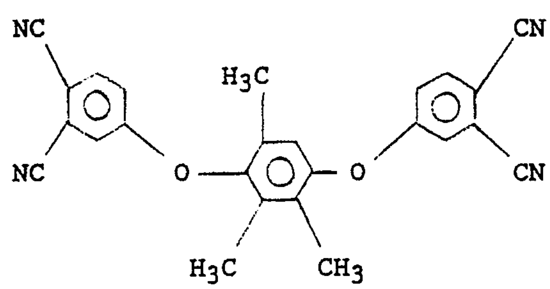

Yield 70% based on the phthalonitrile. The product had a melting point of 287-288°C and elemental analysis, theoretical for $C_{25}H_{16}N_4O_2$: C,74.25%; H,3.96%; N,13.86%; found: C,74.19%; H,3.95%; N,13.95%.

Comparative Experiment B: Preparation of 1,4-bis- (3,4-dicarboxyphenoxy)-2,3,5-trimethyl benzene dianhydride

The tetranitrile prepared according to Comparative Experiment A was converted to the corresponding tetraacid using an equivalent preparation to that of Example 7. The tetraacid was then converted to the corresponding bisetheranhydride, 1,4-bis-(3,4-dicarboxyphenoxy)-2,3,5 trimethyl benzene dianhydride using an equivalent preparation to that in Example 8. This bisetheranhydride has the formula:

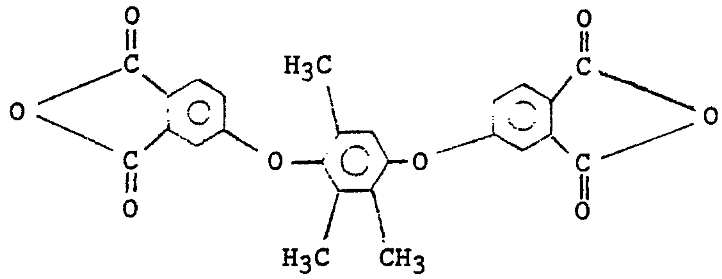

Comparative Experiment C: Preparation of polymer from 4,4′-diaminodiphenylether and 1,4-bis-(3,4 dicarboxyphenoxy)-2,3,5 trimethyl benzene dianhydride

0.002 mol of 4,4′ diaminodiphenyl ether (supplied by Aldrich) was dissolved in 5 ml of dry dimethyl acetamide in a flask fitted with a magnetic stirrer. After dissolution, 0.002 mol of 1,4-bis-(3,4-dicarboxyphenoxy)-2,3,5-trimethyl benzene dianhydride, as prepared in Comparative Experiment B, was added. The solution became very viscous within half an hour and was left to react overnight. A mixture of 0.7 ml of pyridine and 0.7 ml of acetic anhydride was added, after which the viscosity increased and the mixture remained clear, but very viscous. After 6 hours the solution was precipitated into methanol, the fibrous precipitate was filtered off and dried. The dry polymer was redissolved in a small amount of chloroform and reprecipitated into methanol, filtered off and dried. Yield of polymer was over 95%.

Comparative Experiment D: Preparation of polymer from 2,2-bis-(4-aminophenyl) propane and 1,4-bis-(3,4-dicarboxyphenoxy)-2,3,5-trimethyl benzene dianhydride

2.26 g (0.01 mole) of 2,2-bis-(4-aminophenyl) propane was dissolved in 50 ml of anhydrous dimethylacetamide (DMAC) (supplied by Aldrich) in a flask fitted with a magnetic stirrer. 4.44 g (0.01 mole) of 1,4-bis-(3,4-dicarboxyphenoxy)-2,3,5-trimethyl benzene dianhydride was added with stirring at room temperature. The mixture became very viscous after 0.5 hours. To this mixture was added 5 g of anhydrous pyridine and 5 g of acetic anhydride. After standing for six hours the very viscous solution was diluted with 30 ml of DMAC and the polymer was precipitated into methanol and dried.

Comparative Experiment E: Preparation of polymer from 1,4-bis-(3,4-dicarboxyphenoxy)-2,3,5-trimethyl benzene dianhydride and a mixture of 2,2-bis-(4-aminophenyl) propane and 4,4′-diaminodiphenylether)

1.00 g (0.005 mole) of 4,4′-diaminodiphenylether (supplied by Aldrich) and 1.13 g (0.005 mole) of 2,2-bis-(4-aminophenyl) propane were dissolved in 50 ml of anhydrous dimethylacetamide (DMAC) (supplied by Aldrich) in a flask fitted with a magnetic stirrer. 4.44 g (0.01 mole) of 1,4-bis-(3,4-dicarboxyphenoxy)-2,3,5-trimethyl benzene dianhydride was added with stirring at room temperature. After 18 hours 5 g of anhydrous pyridine and 5 g of acetic anhydride were added and the mixture allowed to stand for six hours, during which time a gel was formed. The gel was washed with methanol, dried, and dissolved in 100 ml of chloroform. The solution was poured dropwise into 1000 ml of methanol with vigorous stirring. The polymer was removed by filtration and dried. The yield of polymer was 6.4 g.

Comparative Experiment F: Preparation of bis[4-(3,4-dicyanophenoxy)-3,5-di-tert-butyl-phenyl]methane.

1.73g (10 mmol) of 4-nitrophthalonitrile was dissolved in 15 ml of anhydrous DMSO followed by 2.123 g (0.5 mmol) of 4,4′-methylene-bis-(2,6-di-tert-butylphenol) having the formula:

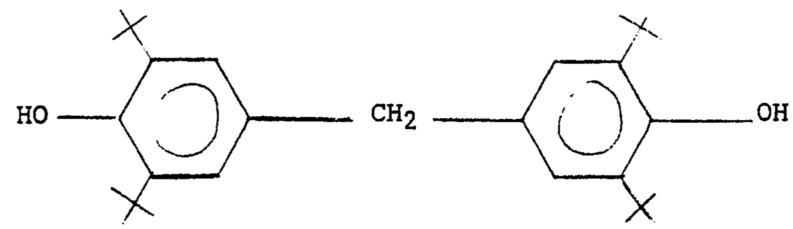

After the material had dissolved, 2.6 g of potassium carbonate was added. The reaction mixture became deep violet in colour and was left, with stirring, for 24 hours. The mixture was then precipitated into 300 ml of water and filtered to yield 3.2 g of a yellow powder readily soluble in methanol and changing colour to deep purple when treated with sodium hydroxide. A similar experiment carried out with oaesium carbonate, instead of potassium carbonate, yielded, after recrystallization, long gold-coloured needles soluble in methanol; the colour again changed when treated with sodium hydroxide. Elemental analysis showed that the product contained 82.17% C and 10.1% H and no nitrogen. We suspect that, because of steric hinderance, the desired nitrodisplacement reaction did not take place.

Comparative Experiment G: Preparation of bis[4-(3,4-dicyanophenoxy)-3-methyl-5-tert-butyl-phenyl]-methane.

4-nitrophthalonitrile (6.92 g, 40 mmol) was dissolved in 40 ml of anhydrous DMSO followed by 6.8 g (20 mmol) of 4,4′-methylene-bis-(2-methyl-6-tert-butylphenol) having the formula:

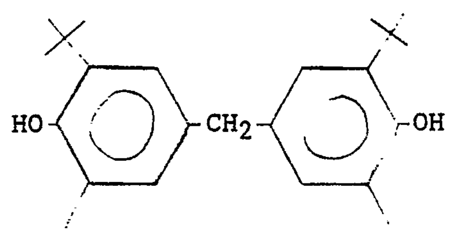

These reagents were reacted as in Example 36 but, again, the attempt to achieve a nitrodisplacement reaction and synthesize a tetranitrile failed. The unidentified, recovered product (20.3 g) (as in Example 36) was readily soluble in methanol and changed colour when treated with sodium hydroxide.

Comparative Experiment H: Preparation of 2,2-bis[4-(3,4-dicyanophenoxy)-3-methyl-5-tert-butyl-phenyl]-propane.

1.2 g (6.3 mmol) of 4-nitrophthalonitrile was dissolved in 15 ml of anhydrous DMSO followed by 1.2 g (3.35 mmol) of 4,4′-dihydroxydiphenyl-[3-methyl-5-tert-butyl]propane having the formula:

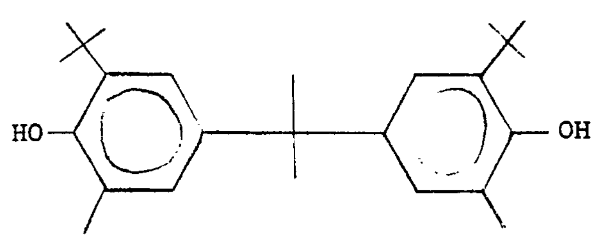

After the ingredients had dissolved, 2.2 g of anhydrous potassium carbonate was added. Reaction was carried out under a nitrogent atmosphere for 24 hours. The mixture was then precipitated into water. 2.0 g of crude tetranitrile was recovered which was recrystallized from 1.5 l of hexane to yield 1.3 g (62.53%) of 2,2-bis-[4-(3,4-dicyanophenoxy)-3-methyl-5-tert-butyl-phenyl] propane having the formula:

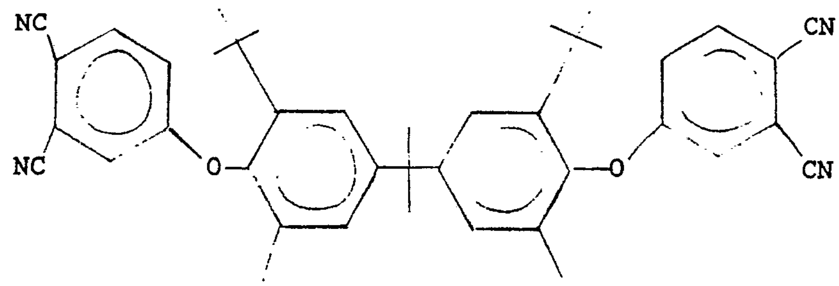

Theoretical analysis of $C_{41}H_{40}N_4O_2$: C,79.35%; H,6.45%; N,9.03%; found; C,79.25%; H,6.78%; N,8.85%.

## Preparation of Membranes

Dense polymer membrane films were prepared using the polymers described in Examples 10 to 15 and 19 to 21 and 25 and Comparative Experiments D and E as follows:

(i) approximately 0.4 g of polymer was dissolved in 20 $cm^3$ of dichloromethane or chloroform.

(ii) the resulting solution was passed through a Millipore prefilter (type AP15) onto a clean glass petri dish.

(iii)the solvent was allowed to evaporate to produce a flexible film which was readily removable from the glass petri dish.

(iv) the polymer membrane films were pretreated at 200 °C in a vacuum oven to remove residual solvent (except Example 15 which was only preheated at 75 °C).

## Gas Permeability Measurements

The constant volume, variable pressure apparatus used to measure gas permeabilities of the membranes is shown in Figures 1 and 2. Figure 1 shows the constant volume, variable pressure apparatus assembled and Figure 2 shows two parts of the apparatus in cross-section and disassembled. The apparatus comprises a cell (14) having a base (1) and a top (2), both of stainless steel. The base has a recess (3) and outlet (4). The recess (3) is capable of receiving a wire mesh support (5), a sample of membrane (6), a rubber gasket (7) and an indium wire sealing ring (8). The top (2) has a gas inlet (10) and is shaped so that it may be assembled with the base to form a gas-tight seal with the membrane and the O-ring (11). The assembled cell may be maintained at a constant temperature by an insulated heat block (12) and is mounted on a frame (13). The inlet (10) is connected to a constant volume receiver (20) (shown schematically) which is provided with means (not shown) for applying a vacuum or for applying gas to the inlet side of the membrane. The outlet (4) is connected to a constant volume receiver (21) (shown schematically) which is provided with means (not shown) for applying vacuum to the outlet side of the membrane. The inlet and outlet are connected to pressure transducers (22) (shown schematically) for measuring pressure.

In use, a sample (6) was cut from a thermally (200 °C for all examples except Example 15 which was treated to only 75 °C) pretreated polymer membrane and placed in the cell (14). The membrane was pretreated in the apparatus overnight by applying vacuum to both the inlet (10) and outlet (4) of the cell with the membrane at 35 °C. Gas (purity>99%) was applied to the inlet side of the membrane at known pressure, and a vacuum was applied to the outlet side of the membrane. The pure gas permeability was determined under steady-state conditions, i.e. gas permeation varying linearly with time where the outlet pressure is negligible compared with the inlet pressure. The pressure on the outlet side of the membrane was measured by the pressure transducer (22) and logged on a computer(not shown). Gas permeabilities were determined for, individual gases, $H_2$, $CO_2$, $O_2$, $N_2$ and $CH_4$ in the applied pressure range 100 to 700 kPa for membranes prepared from polymers prepared according to Examples 10 to 12 and Comparative Experiments D and E are shown in Table 1 and for Examples 13 to 15 and 19 are shown in Table 2 and for Examples 20; 21 and 25 are shown in Table 3. The permeability data shown in Tables 1, 2 and 3 were measured at about 650 kPa.

For Example 15 the apparatus used was similar to that described above except that an O-ring seal was used in place of the indium wire sealing ring (8).

Water Vapour Permeability Measurements

The apparatus used to measure water vapour permeability of polymer membranes prepared according to the present invention is shown schematically in Figure 3.

A sample of the membrane (30) was supported on a sintered disc (not shown) and clamped between two PTFE rings (not shown) in a stainless steel cell (31). The cell (31) was mantained in use at a constant temperature (up to 90 °C) in a temperature-controlled cabinet (32).

Methane gas from a cylinder (33) was passed over one side (34) of the membrane, part of the gas being diverted via a humidifier (35) to provide water vapour to the gas. The composition of the wet methane gas was measured by a gas chromatograph (36).

Helium from a cylinder (37) was passed over the other side (38) of the membrane.

The flow rates of methane and helium were controlled by mass flow controllers (39) and the pressures of the gases on each side of the membrane were measured by pressure indicators (40).

The composition of the helium gas leaving the cell (31) was measured by a gas chromotograph (41).

Membranes were prepared according to Examples 12, 13, 14 and 20 and were anneated at 100 °C in a vacuum oven, except Example 12 which was preheated at 200 °C.

Water vapour permeabilities were measured using these membranes at 30 °C with a feed pressure of 100 psia and the results are shown in Table 4.

Molecular Weight Determination

Molecular weights of the polymer prepared according to the present invention were measured. Molecular weights were measured by use of gel permeation (size exclusion) chromatography. The chromatograph was calibrated using polystyrene standards. The solvent used were tetrahydrofuran or chloroform. The molecular weights quoted are the polystyrene molecular weights corresponding to the elution volume at which the peak of the polyimide chromatogram occurred. The flow rates were 1 ml per minute with an ultra violet detector and the results are shown in Table 5.

Polymer Glass Transition Temperatures and Thermo-oxidative Data

Polymer glass transition temperatures and thermo-oxidative data were measured for polymers prepared according to Examples 10 to 15 and comparative Experiments D and E and the results are shown in Table 6. The glass transition temperatures were measured by differential scanning calorimetry using a Perkin Elmer DSC II instrument.

TABLE 1.

| PERMEABILITY RESULTS AT 35 °C AND 650 kPa | | | | | | | | | |
|---|---|---|---|---|---|---|---|---|---|
| Membrane | Membrane Thickness (micrometres) | Permeability (Barrers) ($cm^3$(STP) cm $cm^{-2}$ $s^{-1}$ $cmHg^{-1}$ x $10^{-10}$) | | | | | Separation factors (ratios of permeabilities) | | |
| | | $P_{CO2}$ | $P_{CH4}$ | $P_{O2}$ | $P_{N2}$ | $P_{H2}$ | $O_2$ $N_2$ | $CO_2$ $CH_4$ | $H_2$ $CH_4$ |
| Example 10 | 40 | 19.0 | 0.874 | 4.51 | 0.914 | 43.3 | 4.9 | 22 | 50 |
| Example 11 | | | | | | | | | |
| Example 12 | 57 | 20.9 | 0.918 | 5.33 | 0.964 | 52.1 | 5.5 | 23 | 57 |
| Comparative Experiment D | 41 | 7.80 | 0.256 | 2.02 | 0.343 | 24.9 | 5.9 | 30 | 97 |
| Comparative Experiment E | 66 | 5.72 | 0.177 | 1.46 | 0.259 | 20.8 | 5.6 | 32 | 118 |

TABLE 2.

| PERMEABILITY RESULTS AT 35° C AND 650 kPa | | | | | | | | | |
|---|---|---|---|---|---|---|---|---|---|
| Membrane | Membrane Thickness (micrometres) | Permeability (Barrers) ($cm^3$(STP) cm $cm^{-2}$ $s^{-1}$ $cmHg^{-1}$ x $10^{-10}$) | | | | | Separation factors (ratios of permeabilities) | | |
| | | $P_{CO2}$ | $P_{CH4}$ | $P_{O2}$ | $P_{N2}$ | $P_{H2}$ | $O_2$ $N_2$ | $CO_2$ $CH_4$ | $H_2$ $CH_4$ |
| Example 13 | 67 | 94.7 | 5.55 | 21.7 | 4.89 | 146.1 | 4.4 | 17 | 26 |
| Example 14 | 36 | 48.9 | 2.58 | 12.0 | 2.46 | 100.2 | 4.9 | 19 | 39 |
| Example 15 | 58 | - | 3.79 | - | - | 69.9 | - | - | 18 |
| Example 19 | 67 | 29.3 | 2.04 | 7.03 | 1.78 | 56.3 | 3.9 | 14 | 28 |

TABLE 3.

| PERMEABILITY RESULTS AT 35° C AND 650 kPa | | | | | | | | | |
|---|---|---|---|---|---|---|---|---|---|
| Membrane | Membrane Thickness (micrometres) | Permeability (Barrers) ($cm^3$(STP) cm $cm^{-2}$ $s^{-1}$ $cmHg^{-1}$ x $10^{-10}$) | | | | | Separation factors (ratios of permeabilities) | | |
| | | $P_{CO2}$ | $P_{CH4}$ | $P_{O2}$ | $P_{N2}$ | $P_{H2}$ | $O_2$ $N_2$ | $CO_2$ $CH_4$ | $H_2$ $CH_4$ |
| Example 20 | 40.6 | - | 6.73 | - | - | 175 | - | - | 26 |
| Example 21 | 78.3 | - | 1.81 | 9.28 | 1.89 | 81.8 | 4.9 | - | 45 |
| Example 25 | 69.4 | 29.5 | 1.51 | 8.06 | 1.47 | 68.9 | 5.5 | 20 | 46 |

TABLE 4

| PERMEABILITY OF WATER VAPOUR | | | | | | |
|---|---|---|---|---|---|---|
| Membrane | Membrane Thickness (Micrometres) | Feed Pressure (psia) | | Permeabilities (Barriers) ($cm^3$(STP)cm $cm^{-2}S^{-1}$cm $Hg^{-1}$x$10^{-10}$) | | Selectivities $H_2O$ $CH_4$ |
| | | Methane | Water | Methane | Water | |
| Example 12 | 25.5 | 99.77 | 0.23 | 1.13 | 3720 | 3292 |
| Example 13 | 21.7 | 99.32 | 0.68 | 7.67 | 4290 | 558 |
| Example 14 | 54.6 | 99.63 | 0.37 | 3.63 | 4430 | 1218 |
| Example 20 | 47.1 | 99.58 | 0.42 | 12.39 | 12537 | 1008 |

Table 5

| Polymer | Peak Molecular Weight/1000* |
|---|---|
| Example 10 | 205 |
| Example 12 | 190 |
| Example 13 | 67 |
| Example 14 | 67 |
| Example 15 | 44 |
| Example 20 | 85 |
| Comparative Experiment D | 141 |
| Comparative Experiment E | 121 |

* Measured in dichloromethane relative to polystyrene standards.

Table 6

| Polymer Thermal and Thermo-oxidative data | | | |
|---|---|---|---|
| Polymer | Tg/°C | T(air)/°C | $T(N_2)$/°C |
| Comparative Example D | 272 | - | - |
| Comparative Example E | 274 | 393 | 412 |
| Example 10 | 254 | - | - |
| Example 12 | 266 | 378 | 465 |
| Example 11 | 251 | - | - |
| Example 14 | 272 | 343 | 412 |
| Example 13 | 322 | 352 | 426 |
| Example 15 | 234 | 250 | 442 |

## Claims

1. A compound having the general formula: X — O — Ar — O — Y
in which X and Y are aromatic mono- or di-basic carboxylic acid groups or derivatives thereof and may be the same or different, and Ar is a divalent aromatic group having at least one tertiary alkyl substituent and not more than one such substituent ortho to each of its bonds to the ether groups of the compound.
2. A compound according to claim 1 in which X and Y has a general formula selected from the group consisting of:

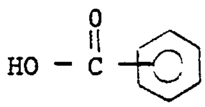 , 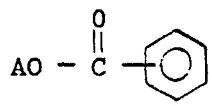 , 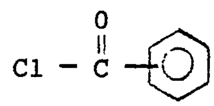

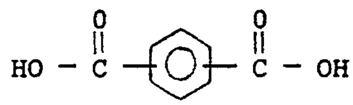 and 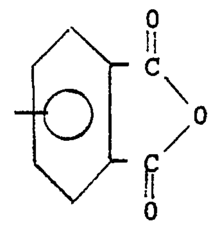

where -A is an alkyl or aryl group having up to 12 carbon atoms.

3. A compound according to claim 1 or claim 2 in which Ar has a general formula selected from the group consisting of:

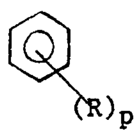 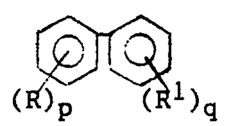 and 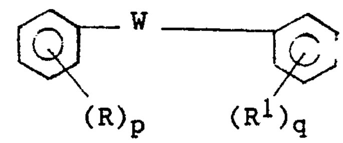

in which -R and $-R^1$ are independently tertiary alkyl groups having up to eight carbon atoms, p and q are independently 0,1,2 or 3, preferably 1 or 2 and p + q is at least one, and -W- is $-CH_2-$, $-C(CH_3)_2-$, $-C(CF_3)_2-$ or

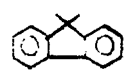

with the proviso that -Ar-does not have more than one tertiary alkyl substituent ortho to eachof its bonds to the ether groups of the compound.

4. A compound according to claim 3 in which R and $R^1$ are tertiary hexyl ($-C(CH_3)_2-(CH_2)-CH_3$) or tertiary pentyl or tertiary butyl groups.

5. A compound according to claim 3 or claim 4 in which Ar has the general formula

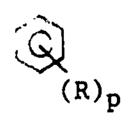

with bonds to the ether groups of the compound which are meta or para positions to each other.

6. A method of making a compound as claimed in any of claims 1 to 5, the method comprising reacting a compound having the general formula:

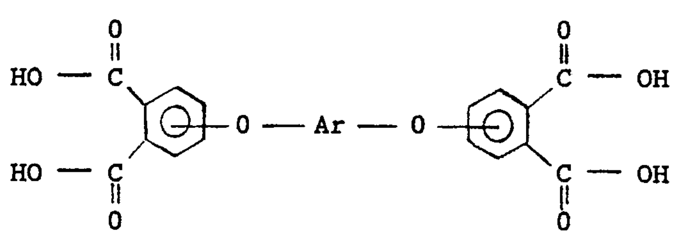

with acetic anhydride at an elevated temperature.

7. A method according to claim 6 in which the product of the reaction is recrystallised with acetic anhydride.

8. A polymer comprising etherimide units having the general formula:

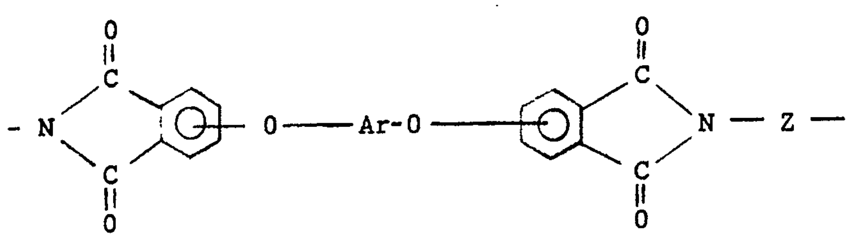

in which -Ar- is as hereinbeforedefined and -Z- is an aromatic divalent group.

9. A polymer according to claim 8 in which a general formula selected from the group consisting of:

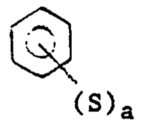

and

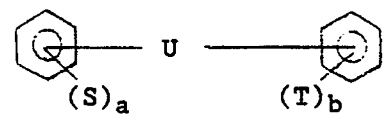

where -S and -T are independently selected from the group consisting of -Cl, -Br, an alkyl group having 1 to 6 carbon atoms and a fluoroalkyl group having 1 to 6 carbon atoms; -S and -T may be the same or different; a and b are independently 0,1,2,3 or 4, for a or b greater than 1 different -S or -T groups may be present in -Z-; and -U- is selected from the group consisting of -0-, -CO-, -CHOH-, -S-, $-SO_2-$. $-CH_2-$, $-C(CH_3)-$, $-C(CF_3)_2-$ and

10. A method of making a polymer comprising etherimide units according to claims 8 and 9, the method comprising reacting a compound having the general formula $H_2N-Z-NH_2$ with a compound having the general formula:

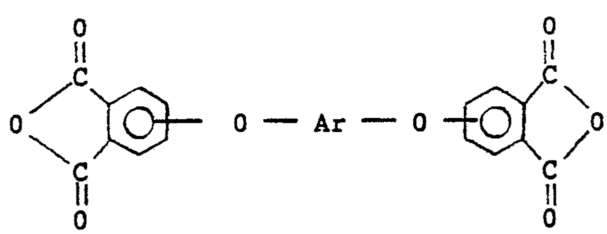

11. A process according to claim 10 in which a mixture of amines of formula $H_2N-Z-NH_2$ are used.

12. A process according to claim 10 or claim 11 in which the reaction is carried out in the presence of pyridine and acetic anhydride.

13. A gas separation membrane comprising a polymer according to claim 8 or claim 9.

14. A gas separation membrane according to claim 13 in which the membrane is in the form of a dense or asymmetric film or in the form of hollow fibres.

15. A process for separating gases, the process comprising passing a mixture of two or more gases through a gas separation membrane as claimed in claim 13 or claim 14 whilst maintaining a differential pressure across the membrane, whereby the gases of the gas mixture are separated by their relative permeation rates through the membrane.

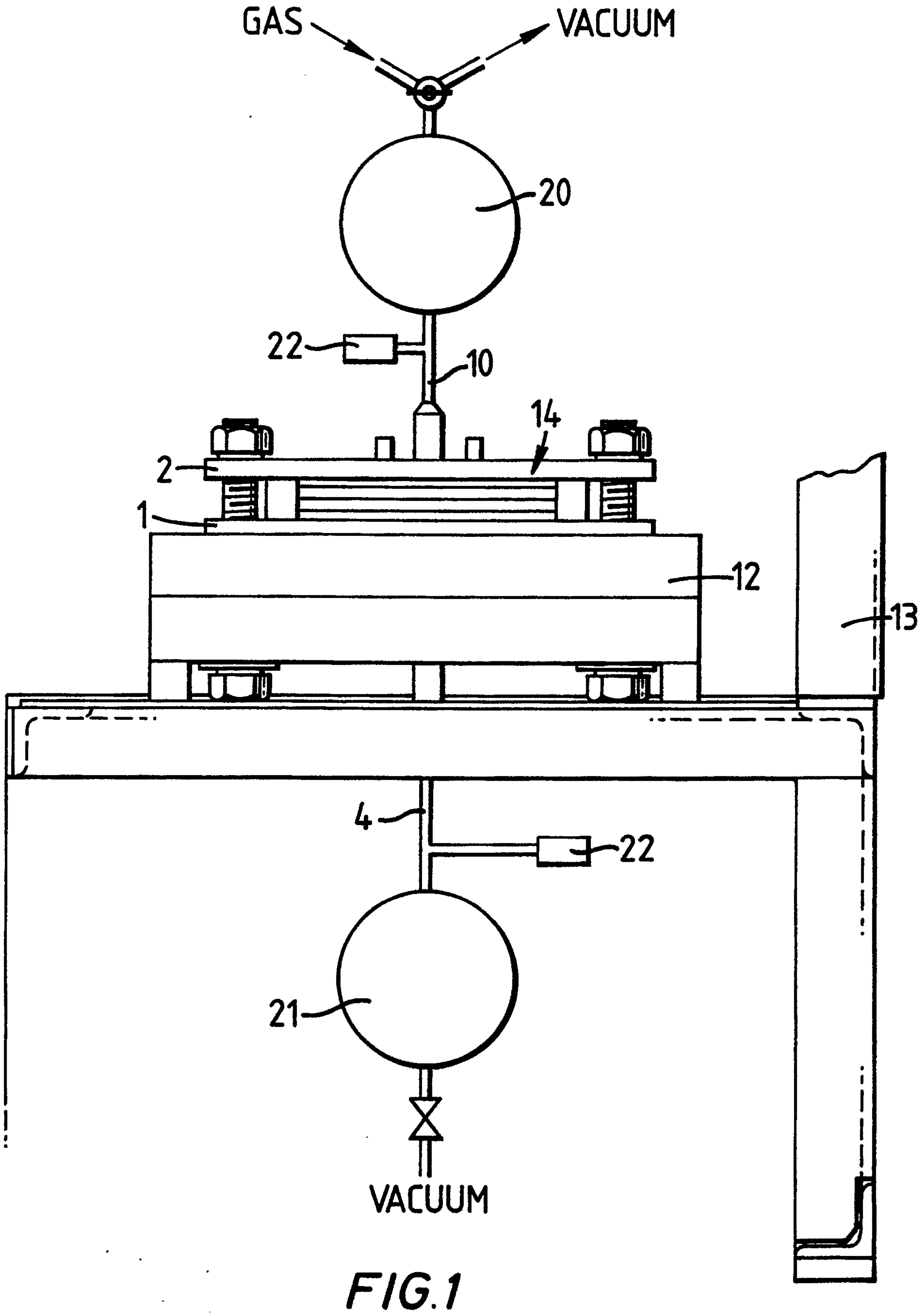


FIG.1

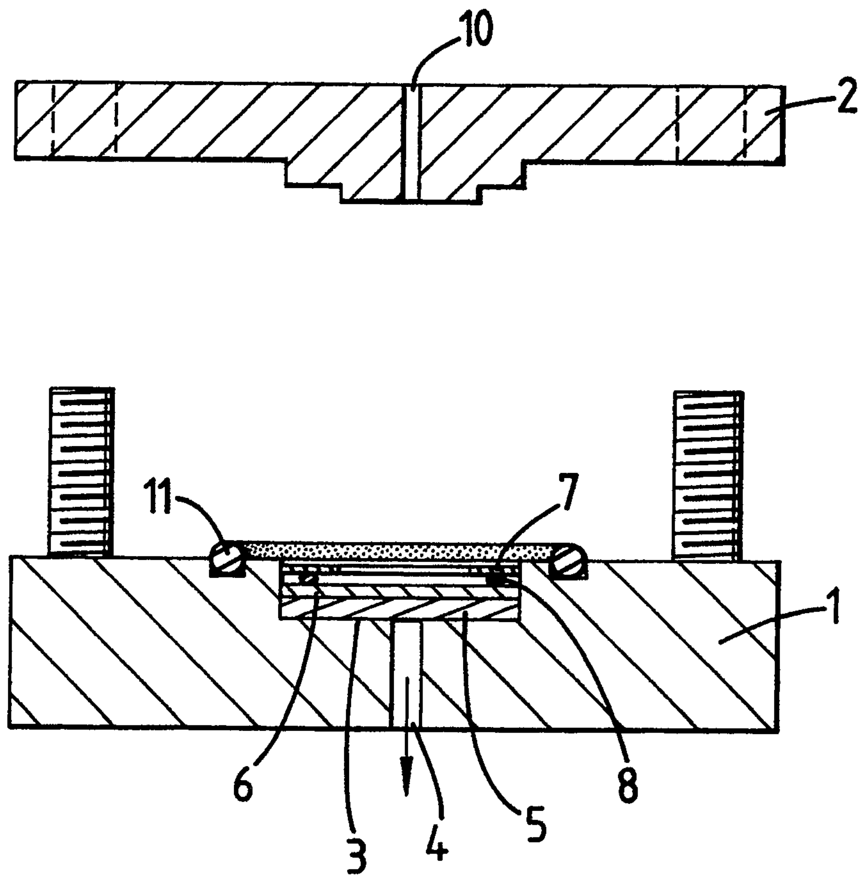


FIG. 2

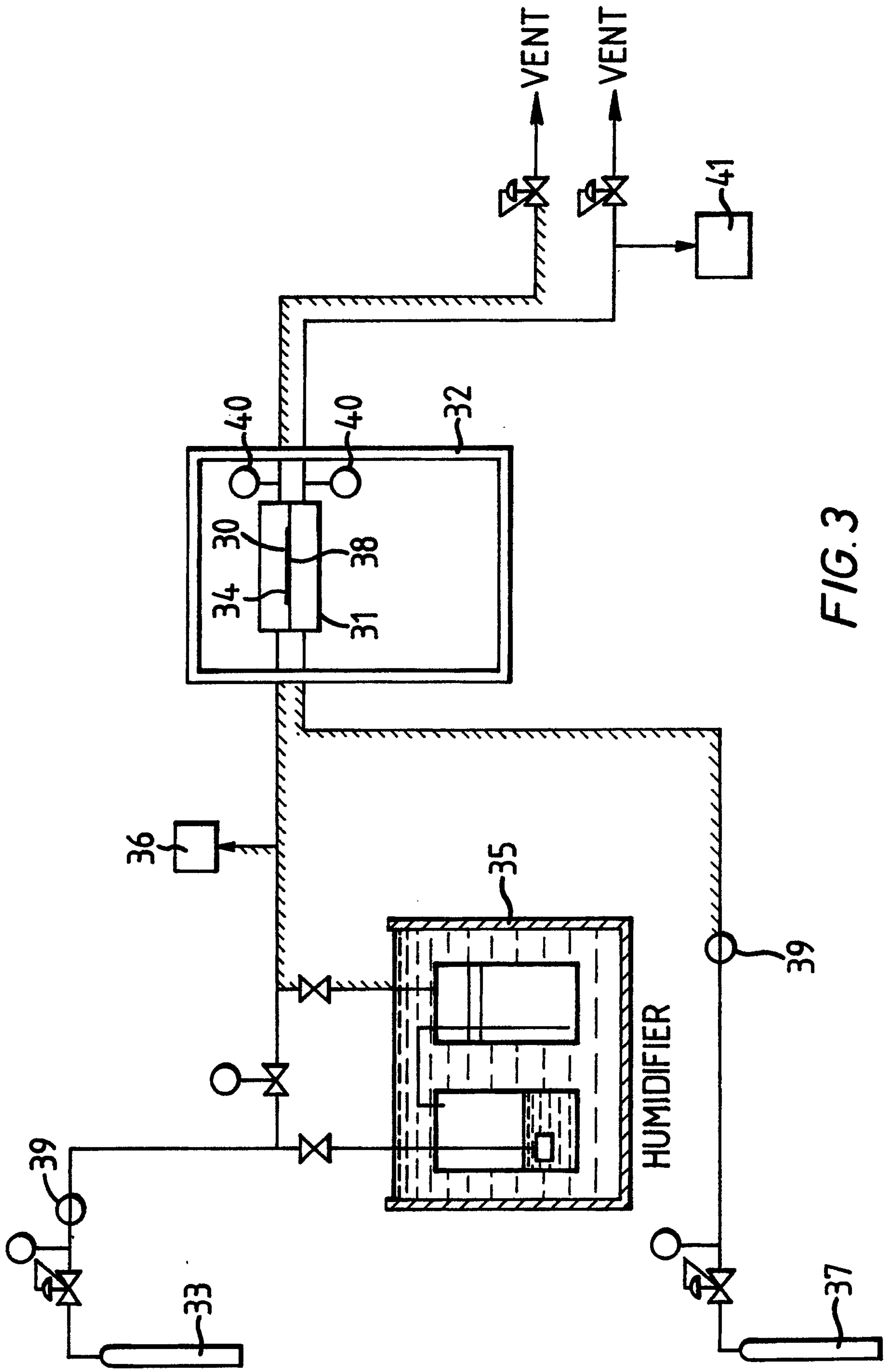


FIG. 3

European Patent Office

# EUROPEAN SEARCH REPORT

Application number: EP 90306306.3

## DOCUMENTS CONSIDERED TO BE RELEVANT

| Category | Citation of document with indication, where appropriate, of relevant passages | Relevant to claim | CLASSIFICATION OF THE APPLICATION (Int. Cl.⁵) |
|---|---|---|---|
| A | DE - A1 - 3 212 163 (GENERAL ELECTRIC) * Claims * -- | 1-3 | C 07 C 65/24<br>C 07 D 307/89<br>C 08 G 73/10<br>B 01 D 71/64 |
| A | EP - A1 - 0 117 416 (GENERAL ELECTRIC) * Claims; examples * -- | 1-3,6, 8,10, 11 | |
| X | US - A - 4 769 476 (P.E. HOWSON) * Column 1, line 58 - column 2 * -- | 1-3,6, 8 | |
| D,A | US - A - 4 717 393 (R.A. HAYES) * Examples * ---- | 8,10, 11,13 | |

TECHNICAL FIELDS SEARCHED (Int. Cl.⁵)

C 07 C 65/00
C 07 D 307/00
C 08 G 73/00
B 01 D 71/00

The present search report has been drawn up for all claims

| Place of search | Date of completion of the search | Examiner |
|---|---|---|
| VIENNA | 11-09-1990 | HOFBAUER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82